# EUROPÄISCHE PATENTSCHRIFT

(11) **EP 4 667 027 B1**
(45) Veröffentlichungstag und Bekanntmachung des Hinweises auf die Patenterteilung: **28.10.2026**
(21) Anmeldenummer: 25181234.3
(22) Anmeldetag: 06.06.2025
(51) Int. Cl.: A61M 1/14

(54) **GEHÄUSE FÜR EIN MEDIZINISCHES GERÄT**
HOUSING FOR A MEDICAL DEVICE
BOÎTIER POUR UN DISPOSITIF MÉDICAL

(30) Priorität: 14.06.2024 DE 102024116858
(43) Veröffentlichungstag der Anmeldung: 24.12.2025
(73) Patentinhaber: B. Braun Avitum AG, 34212 Melsungen (DE)
(72) Erfinder: Schwendich, Wilhelm, 36100 Petersberg (DE); Broeker, Bjoern, 34355 Staufenberg (DE); Moeller, Dirk, 34326 Altmorschen (DE); Schleicher, Christian, 36160 Dipperz (DE)
(74) Vertreter: Patentanwälte Ruff, Wilhelm, Beier, Dauster & Partner mbB

(56) Entgegenhaltungen:
- DE-A1- 102017 111 752
- US-B1- 6 435 727
- US-B2- 9 931 451
- "NIKKISO DBB-05", May 2024 (2024-05-01), Retrieved from the Internet <URL:https://www.ultra-medical.de/angebote/5227853-gebraucht-nikkiso-dbb-05-dialyse-maschine-haemodialyse>

## Beschreibung

Die Erfindung betrifft ein Gehäuse für ein medizinisches Gerät, insbesondere für ein Dialysegerät, sowie ein medizinisches Gerät mit solch einem Gehäuse.

Medizinische Geräte, wie beispielsweise Dialysegeräte, weisen in der Regel eine Vielzahl von am Gehäuse angeordneten Anschlussbuchsen, wie beispielsweise Netzwerkbuchsen und/oder IT-Schnittstellen, auf. Die Anschlussbuchsen sind üblicherweise direkt an einer Außenseite des Gehäuses angeordnet und sind daher nicht oder nur bedingt für die Verwendung in feuchter Umgebung geeignet. Dadurch besteht bei herkömmlichen medizinischen Geräten bei Verwendung in feuchter Umgebung immer das Risiko eines elektrischen Kurzschlusses infolge eingedrungenen Wassers oder Wasserdampfs.

Bei einem unter der Bezeichnung DBB-05 bekannten Dialysegerät des Herstellers Nikkiso ist eine Gehäusewand mit einer Tasche versehen, in welcher eine Anschlussbuchse angeordnet ist.

Aus der US 9 931 451 B2 ist ein Dialysegerät mit einem multifunktionalen Griff bekannt, der als eine Art Vordach für am Gehäuse des Dialysegeräts angeordnete Anschlüsse dienen kann.

Aufgabe der Erfindung ist es, ein Gehäuse für ein medizinisches Gerät bereitzustellen, das Vorteile gegenüber dem Stand der Technik bietet. Insbesondere sollen die Anschlussbuchsen und somit auch das Geräteinnere vor eindringenden Flüssigkeiten, insbesondere vor Spritzwasser und Niederschlag, geschützt sein.

Diese Aufgabe wird durch das Bereitstellen eines Gehäuses mit den Merkmalen von Anspruch 1 gelöst. Die Gehäusewand des erfindungsgemäßen Gehäuses ist mit einer Tasche versehen, die ausgehend von der Außenseite entlang der Längsachse in Richtung einer Innenseite in die Gehäusewand eingesenkt ist. Die Anschlussbuchse ist dabei in der Tasche angeordnet. Hierdurch ist die Anschlussbuchse nicht direkt an der Außenseite der Gehäusewand angeordnet und ist gegenüber Spritzwasser und Niederschlag geschützt. Die Anschlussbuchse kann mit einer im Geräteinneren angeordneten Leiterplatte verbunden sein. Bei einer Ausgestaltung ist die Anschlussbuchse nach unten, in Richtung der Hochachse ausgerichtet. Bei einer weiteren Ausgestaltung ist in der Tasche der Gehäusewand mehr als eine Anschlussbuchse angeordnet. Die Anschlussbuchse kann zusätzlich oder alternativ zum Anschließen einer Energieleitung zum Übertragen von elektrischem Strom und/oder elektrischer Spannung eingerichtet sein. Bei einer weiteren Ausgestaltung ist in der Tasche der Gehäusewand zusätzlich oder alternativ zu der Anschlussbuchse eine elektrische Steckdose vorgesehen. Bei einer Ausgestaltung ist die Tasche als integraler Bestandteil der Gehäusewand ausgebildet und/oder die Gehäusewand weist die Tasche auf. Bei einer weiteren Ausgestaltung ist die Tasche als ein von der Gehäusewand separat gefertigtes Bauteil ausgebildet, insbesondere wobei das Bauteil in eine Aussparung und/oder Durchgangsöffnung der Gehäusewand eingesetzt ist, und/oder das Bauteil ist an der Gehäusewand angeordnet.

Das erfindungsgemäße Gehäuse bietet unterschiedliche Vorteile gegenüber dem Stand der Technik. Insbesondere kann durch die geometrische Gestaltung der Gehäusewand mit der Tasche ein im Vergleich zu etablierten Gestaltungsregeln verbesserter Schutz vor eindringenden Flüssigkeiten in Anschlussbuchsen und in das Geräteinnere erreicht werden.

Weiter gemäß der Erfindung weist die Tasche einen Deckenabschnitt, einen Bodenabschnitt, welcher dem Deckenabschnitt entlang der Hochachse gegenüberliegt, und eine Rückwand auf. Die Rückwand ist entlang der Hochachse und der Querachse zwischen dem Deckenabschnitt und dem Bodenabschnitt flächig erstreckt. Die Anschlussbuchse ist dabei an der Rückwand der Tasche angeordnet. Die Anschlussbuchse ist vorzugsweise in einem oberen Bereich der Rückwand nahe dem Deckenabschnitt angeordnet.

Weiter gemäß der Erfindung ragt der Deckenabschnitt entlang einer Längsachse über die Außenseite der Gehäusewand nach außen und der Deckenabschnitt bildet einen Dachvorsprung. Hierdurch ergibt sich ein weiter verbesserter Schutz gegenüber eindringenden Flüssigkeiten, insbesondere gegenüber Spritzwasser und Niederschlag. Der Dachvorsprung erstreckt sich über mindestens eine gesamte Breite der Tasche. Bei einer Ausgestaltung ragt der Dachvorsprung entlang der Querachse an beiden Seiten über eine gesamte Breite der Rückwand hinaus.

In weiterer Ausgestaltung der Erfindung weist der Dachvorsprung an einer Oberseite eine von der Außenseite der Gehäusewand aus nach unten geneigte Ablauffläche auf. Hierdurch wird ein Ablaufen von Flüssigkeiten auf dem Dachvorsprung vom Geräteinneren weg ermöglicht. Zudem sammelt sich dadurch auf dem Dachvorsprung keine Flüssigkeit an.

Weiter gemäß der Erfindung weist der Dachvorsprung an einer Unterseite eine nach unten geöffnete, entlang der Querachse verlaufende Nut auf. Hierdurch ergibt sich ein weiter verbesserter Schutz gegenüber eindringenden Flüssigkeiten, da die Nut ein Fließen von Flüssigkeit entlang des Deckenabschnitts in Richtung der Rückwand und somit in das Geräteinnere unterbindet, so dass die Flüssigkeit von dem Dachvorsprung nach unten abtropft. Die Nut erstreckt sich vorzugsweise über mindestens die gesamte Breite der Tasche.

In weiterer Ausgestaltung der Erfindung ist die Rückwand der Tasche entlang der Hochachse von unten nach oben und entlang einer Längsachse von innen nach außen geneigt. Die Anschlussbuchse ist dabei vorzugsweise ebenfalls entlang der Hochachse von unten nach oben und entlang einer Längsachse von innen nach außen geneigt. Sollte dennoch Flüssigkeit in die Anschlussbuchse gelangen, fließt hierdurch die Flüssigkeit nach außen in die Umgebung ab. Zusätzlich oder alternativ ist der Deckenabschnitt der Tasche entlang der Längsachse von innen nach außen und entlang der Hochachse von oben nach unten geneigt. Auch hierdurch ergibt sich ein weiter verbesserter Schutz gegenüber eindringenden Flüssigkeiten, da durch die Neigung des Deckenabschnitts die eindringende Flüssigkeit nach außen geleitet wird und somit nicht entlang des Deckenabschnitts in Richtung der Rückwand fließen kann. Zusätzlich oder alternativ ist der Bodenabschnitt der Tasche entlang der Längsachse von innen nach außen und entlang der Hochachse von oben nach unten geneigt. Hierdurch wird auf dem Bodenabschnitt befindliche Flüssigkeit nach außen in die Umgebung geleitet.

In weiterer Ausgestaltung der Erfindung weist die Tasche an einem Randbereich eine Tropfkante auf, wodurch sich Wasser an der Tropfkante sammeln und nach unten abtropfen kann. Bei einer Ausgestaltung erstreckt sich die Tropfkante über mindestens die gesamte Breite der Tasche. Die Tropfkante ist vorzugsweise von der Außenseite der Gehäusewand entlang der Längsachse in Richtung der Umgebung beabstandet.

In weiterer Ausgestaltung der Erfindung ist die Tasche an einem separaten Bauteil ausgebildet. Das separate Bauteil ist in einer Aussparung der Gehäusewand, die zwischen der Außenseite und der Innenseite durchgängig erstreckt ist, angeordnet. Hierdurch ist es in einfacher Art und Weise möglich, das separate Bauteil anzupassen, auszutauschen oder auch in bereits bestehenden medizinischen Geräten nachzurüsten. Zudem vereinfacht das separate Bauteil durch die separate Fertigung nicht nur den Fertigungsprozess, sondern ermöglicht beispielsweise auch durch Spitzguss oder 3D-Druck komplexere Geometrien. Das separate Bauteil ist vorzugsweise mit der Gehäusewand, insbesondere von der Innenseite der Gehäusewand, verschraubt. Bei einer Ausgestaltung ist das separate Bauteil wenigstens abschnittsweise an der Außenseite der Gehäusewand angeordnet.

In weiterer Ausgestaltung der Erfindung ist das separate Bauteil auf der Außenseite der Gehäusewand entlang einer Längsachse abgestützt und die Tasche ragt durch die Aussparung hindurch über die Innenseite der Gehäusewand.

In weiterer Ausgestaltung der Erfindung ist das separate Bauteil aus einem Kunststoffmaterial gefertigt. Das separate Bauteil ist vorzugsweise spritzgegossen. Bei einer Ausgestaltung ist das separate Bauteil spanend gefertigt. Bei einer Ausgestaltung ist das separate Bauteil per additiver Fertigung gefertigt, im Speziellen per 3D-Druck.

Das erfindungsgemäße medizinische Gerät weist ein Gehäuse gemäß der vorhergehenden Beschreibung auf. Im Speziellen handelt es sich bei dem medizinischen Gerät um ein Dialysegerät zur extrakorporalen Blutbehandlung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivansicht eine Ausführungsform eines erfindungsgemäßen medizinischen Geräts in Form eines Dialysegeräts, das eine Ausführungsform eines erfindungsgemäßen Gehäuses aufweist,
- Fig. 2: in teilweise abgeschnittener, schematischer Perspektivansicht das erwähnte Gehäuse des medizinischen Geräts nach Fig. 1 von einer Geräteaußenseite,
- Fig. 3: in teilweise abgeschnittener, schematischer Perspektivansicht das erwähnte Gehäuse des medizinischen Geräts nach Fig. 1 von einer Geräteinnenseite,
- Fig. 4: einen schematischen Längsschnitt des Gehäuses nach Fig. 2 und 3 im Bereich einer Anschlussbuchse,
- Fig. 5: einen vergrößerten Ausschnitt der Fig. 4 im Bereich des Deckenabschnitts,
- Fig. 6: in schematischer Perspektivansicht von schräg unten einen vergrößerten Ausschnitt der Fig. 2,
- Fig. 7: in einer der Fig. 5 entsprechenden Darstellungsweise eine Variante eines erfindungsgemäßen Gehäuses und
- Fig. 8: in einer der Fig. 5 entsprechenden Darstellungsweise eine weitere Variante eines erfindungsgemäßen Gehäuses.

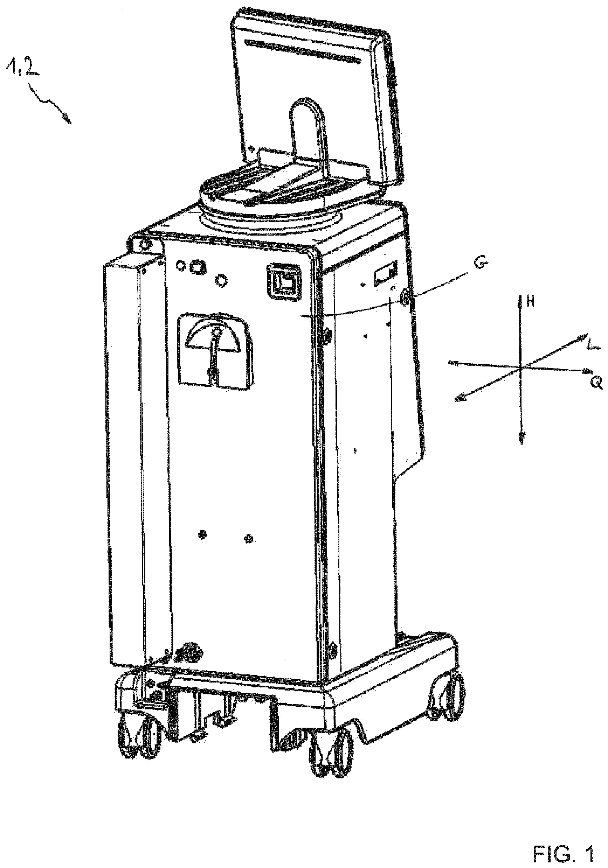

Gemäß Fig. 1 ist ein medizinisches Gerät 1 in Form eines Dialysegeräts 2 zur Verwendung bei einer extrakorporalen Blutbehandlung vorgesehen. Das Dialysegerät 2 weist eine der Fachperson grundsätzlich bekannte Funktion auf, so dass auf diesbezügliche Merkmale des Dialysegeräts 2 nicht weiter eingegangen werden braucht.

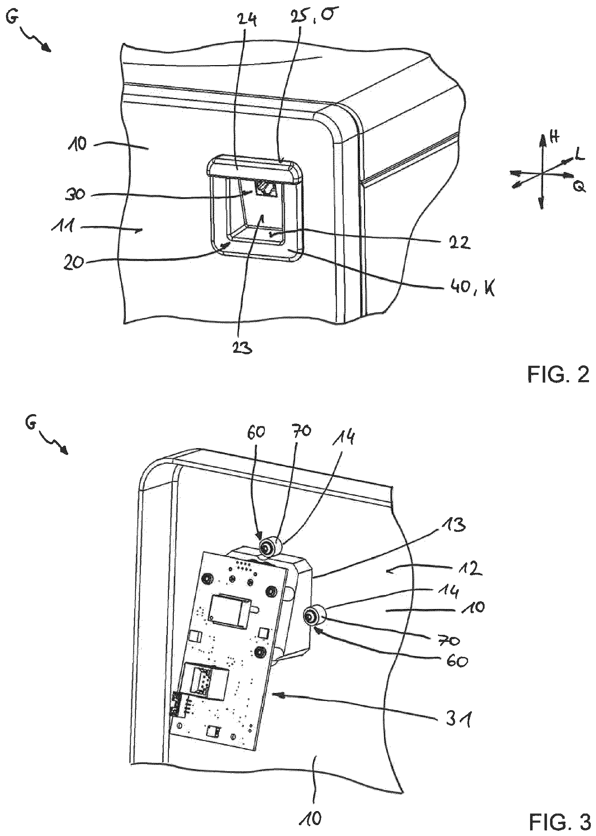

Das Dialysegerät 2 weist ein Gehäuse G auf. Das Gehäuse G weist eine Gehäusewand 10 und eine Anschlussbuchse 30 auf (Fig. 2).

Die Gehäusewand 10 erstreckt sich flächig entlang einer Hochachse H und einer Querachse Q und weist eine der Umgebung zugewandte Außenseite 11 sowie eine Innenseite 12 auf (Fig. 2 und 3). Die Innenseite 12 liegt der Außenseite 11 entlang einer Längsachse L gegenüber. Die Gehäusewand 10 ist mit einer Tasche 20 versehen, die ausgehend von der Außenseite 11 entlang der Längsachse L in Richtung einer Innenseite 12 in die Gehäusewand 10 eingesenkt ist, wie sie im Detail in den Fig. 2 bis 8 gezeigt ist.

Die Anschlussbuchse 30 ist vorliegend zum Anschließen einer Signalleitung und zum Übertragen von Signalen eingerichtet. Bei einer in den Figuren nicht gezeigten Ausführungsform ist die Anschlussbuchse 30 zum Anschließen einer Energieleitung und zum Übertragen von Strom und/oder Spannung eingerichtet.

Die Anschlussbuchse 30 ist in der Tasche 20 angeordnet und ist hierdurch beispielsweise gegenüber Spritzwasser und Niederschlag geschützt.

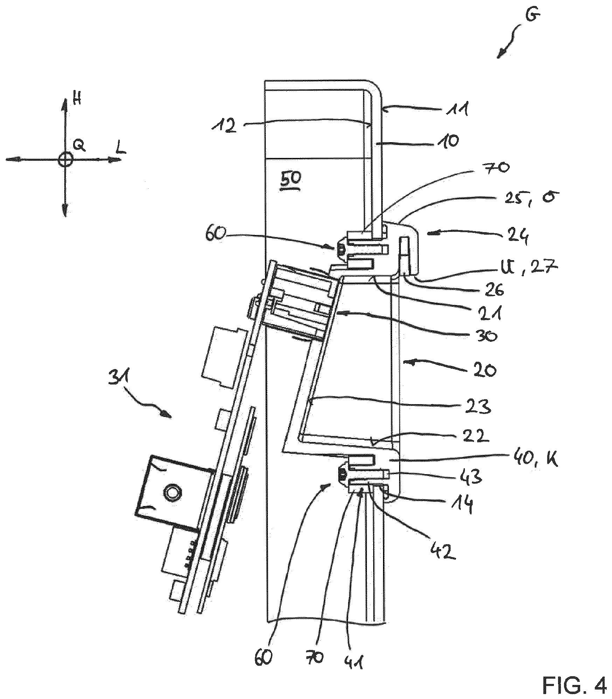

Bei den gezeigten Ausführungen ist die Anschlussbuchse 30 mit einer Leiterplatte 31 verbunden (Fig. 3 und 4).

Bei den gezeigten Ausführungsformen ist am Gehäuse G lediglich eine Tasche 20 mit einer darin angeordneten Anschlussbuchse 30 vorhanden. Die gezeigte Anzahl ist als rein exemplarisch zu verstehen. Bei nicht gezeigten Ausführungen ist das Gehäuse G mit mehreren Taschen 20 versehen. Weiter weist bei nicht gezeigten Ausführungsformen die Tasche 20 mehrere Anschlussbuchsen 30 auf. Nachfolgend werden die weiteren Merkmale des Gehäuses G deshalb unter Bezugnahme auf eine/die Tasche 20 und eine/die Anschlussbuchse 30 erläutert. Für weitere, nicht gezeigte Taschen und Anschlussbuchsen sowie deren Zusammenwirken gilt grundsätzlich das im Hinblick auf die Tasche 20 und die Anschlussbuchse 30 Gesagte sinngemäß.

Bei einer nicht gezeigten Darstellung ist zusätzlich oder alternativ zu der Anschlussbuchse 30 eine elektrische Steckdose vorgesehen.

Vorliegend weist die Tasche 20 einen Deckenabschnitt 21, einen Bodenabschnitt 22 und eine Rückwand 23 auf (Fig. 4). Der Bodenabschnitt 22 liegt dem Deckenabschnitt 21 entlang der Hochachse H gegenüber. Die Rückwand 23 ist entlang der Hochachse H und der Querachse Q zwischen dem Deckenabschnitt 21 und dem Bodenabschnitt 22 flächig erstreckt.

Vorliegend ist die Anschlussbuchse 30 an der Rückwand 23 der Tasche 20 angeordnet (Fig. 2). Dabei ist die Anschlussbuchse 30 in einem oberen Bereich der Rückwand 23 nahe dem Deckenabschnitt 21 angeordnet (Fig. 4).

Bei nicht gezeigten Ausführungen ist die Anschlussbuchse 30 an dem Deckenabschnitt 21 der Tasche 20 angeordnet.

Bei der in den Fig. 1 bis 6 gezeigten Ausführungsform ragt der Deckenabschnitt 21 entlang einer Längsachse L über die Außenseite 11 der Gehäusewand 10 nach außen. Dabei bildet der Deckenabschnitt 21 einen Dachvorsprung 24. Vorliegend ist der Dachvorsprung 24 über eine gesamte Breite B der Tasche 20 erstreckt.

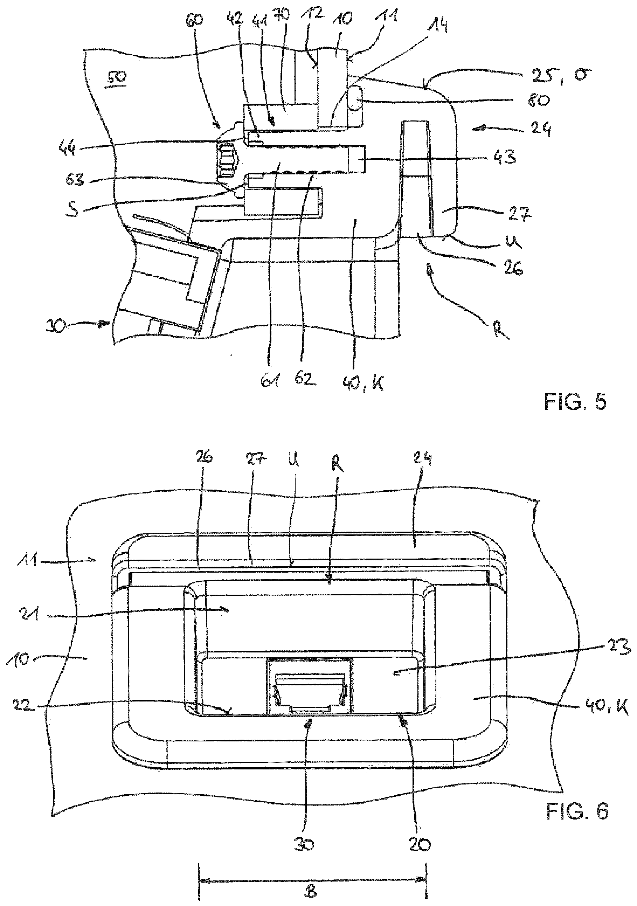

Zudem weist bei dieser Ausführungsform der Dachvorsprung 24 an einer Oberseite O eine von der Außenseite 11 der Gehäusewand 10 aus nach unten geneigte Ablauffläche 25 auf (siehe insbesondere Fig. 5). Dadurch fließt Flüssigkeit von der Gehäusewand 10 nach außen in die Umgebung ab und fließt somit weg vom Geräteinneren 50.

Der in den Fig. 4 bis 6 gezeigte Dachvorsprung 24 weist an einer Unterseite U eine nach unten geöffnete, entlang der Querachse Q verlaufende Nut 26 auf. Vorliegend verläuft die Nut 26 über eine gesamte Breite des Dachvorsprungs 24. Die Nut 26 unterbindet ein Fließen von eindringender Flüssigkeit entlang des Deckenabschnitts 21 in Richtung der Rückwand 23, so dass die Flüssigkeit von dem Dachvorsprung 24 nach unten abtropft. Die Anschlussbuchse 30 ist dabei der Rückwand 23 entsprechend ausgerichtet (Fig. 4).

Bei den gezeigten Ausführungen ist die Rückwand 23 der Tasche 20 entlang der Hochachse H von unten nach oben und entlang einer Längsachse L von innen nach außen geneigt (siehe insbesondere Fig. 4).

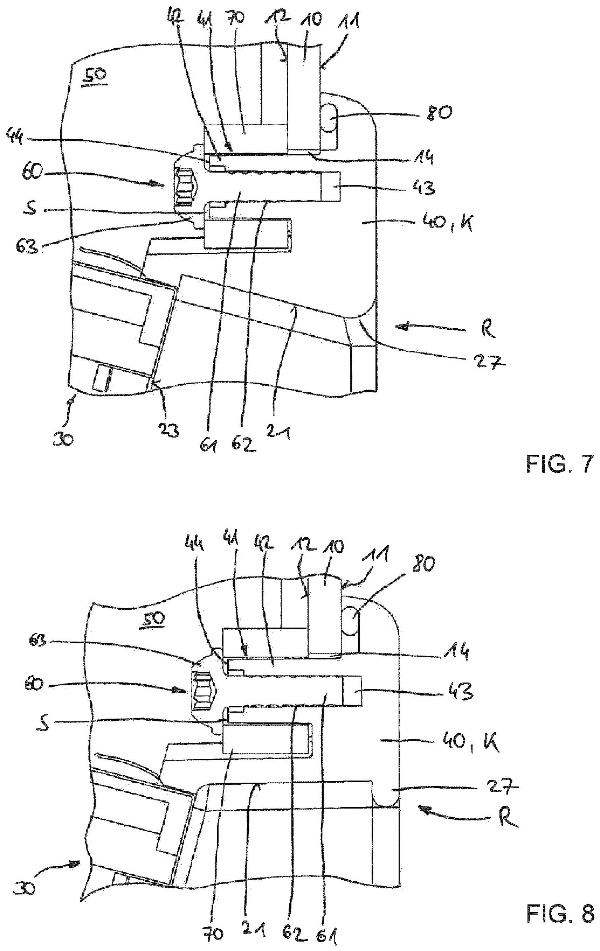

Die Ausführungsform gemäß Fig. 7 zeigt einen Deckenabschnitt 21 der Tasche 20, der entlang der Längsachse L von innen nach außen und entlang der Hochachse H von oben nach unten geneigt ist. Durch diese Neigung des Deckenabschnitts 21 wird eindringende Flüssigkeit nach außen von der Rückwand 23 und somit dem Geräteinneren 50 weg geleitet.

Bei der in Fig. 4 gezeigten Ausführung ist der Bodenabschnitt 22 der Tasche 20 entlang der Längsachse L von innen nach außen und entlang der Hochachse H von oben nach unten geneigt. Durch den geneigten Bodenabschnitt 22 wird ebenfalls eindringende Flüssigkeit nach außen von der Rückwand 23 weg geleitet.

Vorliegend weist die Tasche 20 an einem Randbereich R eine Tropfkante 27 auf, wodurch sich Wasser an der Tropfkante 27 sammeln und nach unten abtropfen kann (Fig. 4 bis 8). Bei der Ausführungsform gemäß der Fig. 2 bis 6 ist die Tropfkante 27 an einer Unterseite U des Dachvorsprungs 24 ausgebildet.

Bei den gezeigten Ausführungen ist die Tasche 20 an einem separaten Bauteil 40 ausgebildet. Das separate Bauteil 40 ist aus einem Kunststoffmaterial K gefertigt. Die Fertigung erfolgt vorliegend per Spritzguss.

Das separate Bauteil 40 ist in einer Aussparung 13 der Gehäusewand 10, die zwischen der Außenseite 11 und der Innenseite 12 durchgängig erstreckt ist, angeordnet (Fig. 2 und 4).

Das separate Bauteil 40 ist auf der Außenseite 11 der Gehäusewand 10 entlang einer Längsachse L abgestützt und die Tasche 20 ragt durch die Aussparung 13 hindurch über die Innenseite 12 der Gehäusewand 10 (Fig. 4).

Vorliegend ist das separate Bauteil 40 von der Innenseite 12 der Gehäusewand 10 mit der Gehäusewand 10 verschraubt. Dazu weist in den gezeigten Ausführungen das separate Bauteil 40 vier Schraubdome 41 mit je einem längserstreckten Domschaft 42 und einer Bohrung 43 auf.

Die Gehäusewand 10 weist zu den Schraubdomen 41 korrespondierende Aussparungen 14 auf, durch welche im montierten Zustand des separaten Bauteils 40 die Schraubdome 41 axial ragen (Fig. 3 und 4). Im Speziellen ragt der Domschaft 42 ausgehend von der Außenseite 11 durch die Aussparung 14 über die Innenseite 12 der Gehäusewand 10. Vorliegend ist bei den gezeigten Ausführungen eine Buchse 70 über ein Stirnende 44 des Domschafts 42 axial auf den Domschaft 42 aufgeschoben und stützt sich axial auf der Innenseite 12 der Gehäusewand 10 ab. Weiter ist eine Schraube 60 vorgesehen, die einen Schraubenschaft 61 mit einem selbstfurchenden Gewinde 62 und einen Schraubenkopf 63 aufweist. Der Schraubenschaft 61 ist in die Bohrung 43 des Schraubdoms 41 eingeschraubt. Beim Einschrauben der Schraube 60 schneidet vorliegend das selbstfurchende Gewinde 62 gleichsam ihr eigenes Gegengewinde in die Bohrung 43. Der Schraubenkopf 63 ist axial auf der Buchse 70 abgestützt. Die Buchse 70 ragt axial über das Stirnende 44 des Domschafts 42 hinaus (siehe Fig. 5, 7 und 8). Hierdurch ist zwischen dem Schraubenkopf 63 und dem Stirnende 44 des Domschafts 42 ein Axialspalt S ausgebildet. Der Axialspalt S gewährleistet einen vorteilhaften Kraftfluss über die Buchse 70. Dabei werden der Schraubenschaft 61 und der Domschaft 42 axial auf Zug beansprucht und der Schraubenkopf 63 drückt axial auf die Buchse 70, die wiederum axial auf der Innenseite 12 der Gehäusewand 10 abgestützt ist. Hierdurch wird das separate Bauteil 40 axial gegen die Außenseite 11 der Gehäusewand 10 gezogen/gepresst.

Im Übrigen ist bei den gezeigten Ausführungsformen ein Dichtelement 80 vorgesehen, das als optional zu verstehen ist. Das Dichtelement 80 ist zwischen der Außenseite 11 der Gehäusewand 10 und einer nicht näher bezeichneten Innenseite des separaten Bauteils 40 angeordnet. Beim Anziehen der Schraubverbindung wird das Dichtelement 80 komprimiert und/oder gequetscht.

Bei in den Figuren nicht gezeigten Ausführungsformen ist die Tasche 20 als ein integraler Bestandteil der Gehäusewand 10 ausgebildet.

## Patentansprüche

1. Gehäuse (G) für ein medizinisches Gerät (1), insbesondere ein Dialysegerät (2), aufweisend
eine Gehäusewand (10), die sich entlang einer Hochachse (H) und einer Querachse (Q) flächig erstreckt und eine der Umgebung zugewandte Außenseite (11) sowie eine Innenseite (12) aufweist, wobei die Innenseite (12) der Außenseite (11) entlang einer Längsachse (L) gegenüberliegt,
eine Anschlussbuchse (30), die zum Anschließen einer Signalleitung und zum Übertragen von Signalen eingerichtet ist,
wobei die Gehäusewand (10) mit einer Tasche (20) versehen ist, die ausgehend von der Außenseite (11) entlang der Längsachse (L) in Richtung einer Innenseite (12) in die Gehäusewand (10) eingesenkt ist,
wobei die Anschlussbuchse (30) in der Tasche (20) angeordnet und hierdurch gegenüber Spritzwasser geschützt ist,
wobei die Tasche (20) einen Deckenabschnitt (21), einen Bodenabschnitt (22), welcher dem Deckenabschnitt (21) entlang der Hochachse (H) gegenüberliegt, und eine Rückwand (23) aufweist, die entlang der Hochachse (H) und der Querachse (Q) zwischen dem Deckenabschnitt (21) und dem Bodenabschnitt (22) flächig erstreckt ist, wobei die Anschlussbuchse (30) an der Rückwand (23) der Tasche (20) angeordnet ist,
wobei der Deckenabschnitt (21) entlang einer Längsachse (L) über die Außenseite (11) der Gehäusewand (10) nach außen ragt und einen Dachvorsprung (24) bildet, welcher sich über mindestens eine gesamte Breite (B) der Tasche (20) erstreckt,
dadurch charakterisiert, dass der Dachvorsprung (24) an einer Unterseite (U) eine nach unten geöffnete, entlang der Querachse (Q) verlaufende Nut (26) aufweist.

2. Gehäuse (G) nach Anspruch 1, wobei der Dachvorsprung (24) an einer Oberseite (O) eine von der Außenseite (11) der Gehäusewand (10) aus nach unten geneigte Ablauffläche (25) aufweist.

3. Gehäuse (G) nach einem der vorhergehenden Ansprüche, wobei
die Rückwand (23) der Tasche (20) entlang der Hochachse (H) von unten nach oben und entlang einer Längsachse (L) von innen nach außen geneigt ist, und/oder
der Deckenabschnitt (21) der Tasche (20) entlang der Längsachse (L) von innen nach außen und entlang der Hochachse (H) von oben nach unten geneigt ist, und/oder
der Bodenabschnitt (22) der Tasche (20) entlang der Längsachse (L) von innen nach außen und entlang der Hochachse (H) von oben nach unten geneigt ist.

4. Gehäuse (G) nach einem der vorhergehenden Ansprüche, wobei die Tasche (20) an einem Randbereich (R) eine Tropfkante (27) aufweist, wodurch sich Wasser an der Tropfkante (27) sammeln und nach unten abtropfen kann.

5. Gehäuse (G) nach einem der vorhergehenden Ansprüche, wobei die Tasche (20) an einem separaten Bauteil (40) ausgebildet ist, welches in einer Aussparung (13) der Gehäusewand (10), die zwischen der Außenseite (11) und der Innenseite (12) durchgängig erstreckt ist, angeordnet ist und vorzugsweise mit der Gehäusewand (10) verschraubt ist.

6. Gehäuse (G) nach Anspruch 5, wobei das separate Bauteil (40) auf der Außenseite (11) der Gehäusewand (10) entlang einer Längsachse (L) abgestützt ist und die Tasche (20) durch die Aussparung (13) hindurch über die Innenseite (11) der Gehäusewand (10) ragt.

7. Gehäuse (G) nach Anspruch 5 oder 6, wobei das separate Bauteil (40) aus einem Kunststoffmaterial (K) gefertigt, insbesondere spritzgegossen, ist.

8. Medizinisches Gerät (1), insbesondere Dialysegerät (2), mit einem Gehäuse (G) nach einem der vorhergehenden Ansprüche.

## Claims

1. Housing (G) for a medical appliance (1), in particular a dialysis appliance (2), having
a housing wall (10) which extends flat along a vertical axis (H) and a transverse axis (Q) and has an outer side (11) facing the surroundings and an inner side (12), the inner side (12) lying opposite the outer side (11) along a longitudinal axis (L),
a connection socket (30), which is configured for the connection of a signal line and for the transmission of signals,
wherein the housing wall (10) is provided with a pocket (20) which is recessed into the housing wall (10) from the outer side (11) along the longitudinal axis (L) in the direction of an inner side (12),
wherein the connection socket (30) is located in the pocket (20) and is thereby protected against spray,
wherein the pocket (20) has a top portion (21), a bottom portion (22), which lies opposite the top portion (21) along the vertical axis (H), and a rear wall (23) which extends flat along the vertical axis (H) and the transverse axis (Q) between the top portion (21) and the bottom portion (22), wherein the connection socket (30) is located on the rear wall (23) of the pocket (20),
wherein the top portion (21) projects outwards over the outer side (11) of the housing wall (10) along a longitudinal axis (L) and forms a roof projection (24), which extends over at least an entire width (B) of the pocket (20),
**characterized in that** the roof projection (24) has, on an underside (U), a groove (26) which is downwardly open and runs along the transverse axis (Q).

2. Housing (G) according to Claim 1, wherein the roof projection (24) has, on an upper side (O), a drainage surface (25) which is downwardly inclined outwards from the outer side (11) of the housing wall (10).

3. Housing (G) according to either of the preceding claims, wherein
the rear wall (23) of the pocket (20) is inclined along the vertical axis (H) from the bottom upwards and along a longitudinal axis (L) from the inside outwards, and/or
the top portion (21) of the pocket (20) is inclined along the longitudinal axis (L) from the inside outwards and along the vertical axis (H) from the top downwards, and/or
the bottom portion (22) of the pocket (20) is inclined along the longitudinal axis (L) from the inside outwards and along the vertical axis (H) from the top downwards.

4. Housing (G) according to any one of the preceding claims, wherein the pocket (20) has a drip edge (27) at an edge region (R), as a result of which water can collect at the drip edge (27) and drip downwards.

5. Housing (G) according to any one of the preceding claims, wherein the pocket (20) is formed on a separate component (40) which is located in a cutout (13) of the housing wall (10), which extends continuously between the outer side (11) and the inner side (12), and is preferably screwed to the housing wall (10).

6. Housing (G) according to Claim 5, wherein the separate component (40) is supported on the outer side (11) of the housing wall (10) along a longitudinal axis (L), and the pocket (20) projects through the cutout (13) over the inner side (11) of the housing wall (10).

7. Housing (G) according to Claim 5 or 6, wherein the separate component (40) is manufactured, in particular injection-moulded, from a plastics material (K).

8. Medical appliance (1), in particular dialysis appliance (2), having a housing (G) according to any one of the preceding claims.

## Revendications

1. Boîtier (G) pour un dispositif médical (1), en particulier un appareil de dialyse (2), comportant
une paroi (10) de boîtier, qui s'étend à plat le long d'un axe vertical (H) et d'un axe transversal (Q) et qui comporte un côté extérieur (11) tourné vers l'environnement ainsi qu'un côté intérieur (12), le côté intérieur (12) faisant face au côté extérieur (11) le long d'un axe longitudinal (L),
une prise de raccordement (30), qui est mise au point pour raccorder une ligne de signaux et pour transmettre des signaux,
la paroi (10) de boîtier étant pourvue d'un compartiment (20) qui est enfoncé dans la paroi (10) de boîtier en partant du côté extérieur (11) le long de l'axe longitudinal (L) en direction d'un côté intérieur (12),
la prise de raccordement (30) étant disposée dans le compartiment (20) et étant ainsi protégée contre les projections d'eau,
le compartiment (20) comportant une section de plafond (21), une section de fond (22), qui fait face à la section de plafond (21) le long de l'axe vertical (H), et une paroi arrière (23), qui s'étend à plat le long de l'axe vertical (H) et de l'axe transversal (Q) entre la section de plafond (21) et la section de fond (22), la prise de raccordement (30) étant disposée sur la paroi arrière (23) du compartiment (20),
la section de plafond (21) dépassant vers l'extérieur le long d'un axe longitudinal (L) sur le côté extérieur (11) de la paroi (10) de boîtier et formant une partie faisant saillie de toit (24), qui s'étend sur au moins toute la largeur (B) du compartiment (20),
**caractérisé en ce que** la partie faisant saillie de toit (24) comporte, sur un côté inférieur (U), une rainure (26) ouverte vers le bas, s'étendant le long de l'axe transversal (Q).

2. Boîtier (G) selon la revendication 1, la partie faisant saillie de toit (24) comportant, sur un côté supérieur (O), une surface d'écoulement (25) inclinée vers le bas à partir du côté extérieur (11) de la paroi (10) de boîtier.

3. Boîtier (G) selon l'une des revendications précédentes,
la paroi arrière (23) du compartiment (20) étant inclinée du bas vers le haut le long de l'axe vertical (H) et de l'intérieur vers l'extérieur le long d'un axe longitudinal (L), et/ou
la section de plafond (21) du compartiment (20) étant inclinée de l'intérieur vers l'extérieur le long de l'axe longitudinal (L) et du haut vers le bas le long de l'axe vertical (H), et/ou
la section de fond (22) du compartiment (20) étant inclinée de l'intérieur vers l'extérieur le long de l'axe longitudinal (L) et du haut vers le bas le long de l'axe vertical (H).

4. Boîtier (G) selon l'une des revendications précédentes, le compartiment (20) comportant un larmier (27) sur une zone de bord (R), laissant ainsi à l'eau la possibilité de s'accumuler sur le larmier (27) et de s'égoutter vers le bas.

5. Boîtier (G) selon l'une des revendications précédentes, le compartiment (20) étant formé sur un composant séparé (40), qui est disposé dans un évidement (13) de la paroi (10) de boîtier, qui s'étend en continu entre le côté extérieur (11) et le côté intérieur (12) et qui est vissé de préférence sur la paroi (10) de boîtier.

6. Boîtier (G) selon la revendication 5, le composant séparé (40) étant supporté sur le côté extérieur (11) de la paroi (10) de boîtier le long d'un axe longitudinal (L) et le compartiment (20) dépassant à travers l'évidement (13) au-delà du côté intérieur (11) de la paroi (10) de boîtier.

7. Boîtier (G) selon la revendication 5 ou 6, le composant séparé (40) étant fabriqué, en particulier moulé par injection, à partir d'un matériau en matière plastique (K).

8. Dispositif médical (1), en particulier appareil de dialyse (2), avec un boîtier (G) selon l'une des revendications précédentes.
